# EUROPEAN PATENT SPECIFICATION

(11) **EP 3 653 735 B1**
(45) Date of publication and mention of the grant of the patent: **13.10.2021**
(21) Application number: 18832705.0
(22) Date of filing: 15.06.2018
(51) Int. Cl.: C14C 11/00, A44C 5/02, A44C 5/10, C14C 3/06, C07C 39/14, C08G 18/42, C08G 18/72, C08G 18/73, C08G 18/75, C08G 18/76, C09D 101/18, C09D 175/06

(54) **REPTILE SKIN PRODUCT AND PRODUCTION METHOD FOR REPTILE SKIN PRODUCT**
REPTILIENHAUTPRODUKT UND VERFAHREN ZUR HERSTELLUNG EINES REPTILIENHAUTPRODUKTS
PRODUIT DE PEAU DE REPTILE ET SON PROCÉDÉ DE PRODUCTION

(30) Priority: 13.07.2017 JP 2017137038
(43) Date of publication of application: 20.05.2020
(73) Proprietor: Citizen Watch Co., Ltd., Tokyo 188-8511 (JP)
(72) Inventor: AKAO, Yuji, Nishitokyo-shi Tokyo 188-8511 (JP)
(74) Representative: Hoffmann Eitle
(86) International application number: PCT/JP2018/022925
(87) International publication number: WO 2019/012913

(56) References cited:
- EP-A1- 3 178 947
- JP-A- S4 835 001
- JP-A- S63 199 800
- JP-A- 2017 039 876
- US-A- 3 834 936
- DATABASE WPI Week 201718 Thomson Scientific, London, GB; AN 2017-14168Y XP002801219, & JP 2017 039876 A (JAPAN INST LEATHER RES) 23 February 2017 (2017-02-23)

## Description

### Field

The present invention relates to a reptile leather article and a manufacturing method therefor. Background

In conventional leather watchbands, a surface layer containing nitrocellulose or the like is provided on the surface of the watchband body (Patent Literature 1). In particular, a surface layer containing nitrocellulose is often provided in a reptile leather watchband body to impart a luster.

Furthermore, US 3,834,936 is concerned with a process for dressing leather and artificial leather by applying thereto solutions in organic solvents of combinations of (1) polyurethanes with terminal hydroxyl groups in the molecular weight range of 5,000 to 40,000, prepared by reacting linear or slightly branched polyesters in the molecular weight range of 500 to 5,000 which have terminal hydroxyl groups with 3-isocyanato-3,5,5-trimethylcyclohexylisocyanate; (2a) 15 to 100% by weight, based on polyurethane, of vinyl chloride copolymers in the molecular weight range of 25,000 to 150,000 consisting of 70 to 99.5% by weight of vinyl chloride units, 0 to 30% by weight of vinyl alcohol units and/or 0 to 30% by weight of vinyl acetate units and/or 0 to 30% by weight of units corresponding to the copolymerization of α,β-unsaturated mono- or dicarboxylic acids or their esters or semiesters and 0 to 30% by weight, based on polyurethane, of nitrocellulose which may contain plasticizer, or (2b) 30 to 130% by weight, based on polyurethane, of nitrocellulose which may contain plasticizer and optionally 0 to 15% by weight, based on polyurethane, of vinyl chloride copolymers of the type mentioned under (2a); and (3) 0 to 30% by weight, based on polyurethane, of polyisocyanates.

### Citation List

### Patent Literature

Patent Literature 1: Japanese Examined Patent Publication No. S61-20281

### Summary

### Technical Problem

The surface layer presents the problem of easy peeling. Furthermore, a protective film is pasted onto the watchband buckle so that damage is not sustained between, for example, the time of manufacturing and the time of sale. This protective film may cover a portion of the watchband body as well as the buckle. In that case, a portion of the surface layer containing the nitrocellulose easily peels off along with the protective film when the protective film is peeled off at the time of sale or the like. The aesthetics of the watchband may be thereby impaired.

Even if a reptile leather article other than a watchband has a metal or other such part (e.g., a fastener or a decorative part), a protective film may be pasted onto the part between, for example, the time of manufacturing and the time of sale. Therefore, the problem may occur also to reptile leather articles other than a watchband.

Accordingly, it is an object of the present invention to provide a reptile leather article capable of suppressing peeling of the surface layer containing nitrocellulose and a manufacturing method therefor. Solution to Problem

The object is solved by the reptile leather article and the manufacturing method according to the present invention as defined in the appended claims.

The reptile leather article according to the present invention includes an intermediate layer containing polyester polyurethane and a surface layer containing nitrocellulose laminated, in that order, on a grain side of reptile leather.

### Advantageous Effects of Invention

The reptile leather article of the present invention suppresses peeling of the surface layer containing nitrocellulose.

### Description of Embodiments

Modes (embodiments) for working the present invention are described in detail below.

### <First embodiment>

A watchband that is a reptile leather article of a first embodiment is described. The watchband has a watchband body and, for attachment to the arm, a metal or other such fastener. Examples of the fastener include a buckle and an inner clasp. In the first embodiment, an intermediate layer containing polyester polyurethane and a surface layer containing nitrocellulose are laminated, in that order, on the grain side of reptile leather composing the watchband body.

Examples of reptile leathers include the leathers of reptiles such as sea turtles in the Cheloniidae family of the Testudines order, monitor lizards in the Varanidae family of the Lacertilia suborder, the degu in the Teeiddo family of the Lacertilia suborder, the reticulated python in the Boidae family of the Serpentes suborder, the Indian python, sea snakes in the Hydrophiidae family of the Serpentes suborder, the black-banded sea krait, water snakes in the Hebi family of the Serpentes suborder, the New Guinea crocodile in the Crocodylidae family of the Crocodilia order, the American alligator in the Alligatoridae family of the Crocodilia order, and the caiman. From an aesthetic viewpoint, a small type selected from among the species composing the Crocodylidae family of the Crocodilia order and the Alligatoridae family of the Crocodilia order are used particularly suitably as a watchband.

Such a reptile leather can be obtained with a known method. An example of such a method is to carry out a preparation step (soaking, liming/unhairing, reliming, deliming/bating and the like), a chrome tanning step, a dyeing step, a fatliquoring step, and a finishing step. Then a treatment such as glazing for bringing out a luster is carried out on the grain side of the reptile leather as the finishing step.

More specifically, in the first embodiment, an intermediate layer containing polyester polyurethane is provided on the grain side of the reptile leather treated to bring out the luster. Further the surface layer containing nitrocellulose is provided on the intermediate layer on the grain side of the reptile leather to further bring out the luster.

A specific example of the watchband body is pig leather further laminated, via a cushion layer, a core material layer or the like, on the side (flesh side) opposite of the grain side of the reptile leather provided with the surface layer. The watchband body is formed into a shape and size ordinarily used as a watchband. If a buckle is used as the fastener, a hole is made for inserting the tang into the watchband body.

A protective film is ordinarily pasted onto the fastener of the watchband so that damage is not sustained between, for example, the time of manufacturing and the time of sale. An example of the protective film is a film formed by providing an acrylic adhesive layer on a base material. Polyethylene or the like may be used as the base material. The protective film may cover a portion of the watchband body (grain side of the reptile leather) as well as the fastener.

In conventional watchbands, a portion of the surface layer containing nitrocellulose may peel along with the protective film when the protective film is peeled off at, for example, the time of sale. That is to say, the aesthetics of the watchband may be impaired. In conventional watchbands, it is thought that such impairment is due to direct lamination of the surface layer on the grain side of the reptile leather with a low adhesiveness between the reptile leather and the surface layer. A fatliquoring agent is added to the reptile leather at the time of manufacturing. It is thought that the fatliquoring agent may shift to the surface side of the reptile leather due to heat generated during glazing. The shifted fatliquoring agent is thought to further reduce the adhesiveness between the reptile leather and the surface layer.

In contrast, the watchband that is the reptile leather article of the first embodiment suppresses peeling of the surface layer containing nitrocellulose when the protective film is peeled off at, for example, the time of sale. That is to say, the aesthetics of the watchband can be maintained. This is because an intermediate layer containing polyester polyurethane is provided between the grain side of the reptile leather and the surface layer. More specifically, it is thought that the aesthetics of the watchband can be maintained because the urethane bond in the polyester polyurethane has a high affinity with the polypeptide bond in the proteins composing the reptile leather. It is also thought that the urethane bond having a high affinity with the polar portion of the nitrocellulose contained in the surface layer makes a contribution. It is thought that, in this manner, the adhesion between the reptile leather and the surface layer increases through the intermediate layer.

Furthermore, in the first embodiment, the peeling of the surface layer is suppressed more than when polyether polyurethane is used. Unlike polyether polyurethane, polyester polyurethane has an ester group. It is thought that this ester group further enhances the adhesion to the reptile leather even in the presence of a fatliquoring agent that floats to the surface. It is also thought that the presence of the ester group increases the affinity with the polar portion of the nitrocellulose contained in the surface layer.

The use of polyester polyurethane enhances the colorless transparency of the intermediate layer more than the use of polyether polyurethane. Furthermore, providing polyester polyurethane in the intermediate layer is preferable to providing polyester and polyurethane separately in the intermediate layer from the perspective of solvent selection when forming the intermediate layer.

The intermediate layer and the surface layer are described in detail. The intermediate layer contains polyester polyurethane. One type of polyester polyurethane may be used alone or two or more used in combination. The polyester polyurethane is obtained by causing a polyester polyol, which is a polyol component, and a polyisocyanate component to react. Therefore, the polyester polyurethane has a constituent unit derived from polyester polyol and a constituent unit derived from a polyisocyanate component.

Examples of the polyester polyol include a polycaprolactone polyol and a polyester polyol comprising a polycondensate of an alcohol such as ethylene glycol, diethylene glycol, propylene glycol, butylene glycol, hexamethylene glycol, 1,4-butanediol, 1,6-hexanediol, 1,9-nonanediol, 2-methyl-1,3-propanediol, neopentyl glycol, or 3-methyl-1,5-pentanediol and a dibasic acid such as adipic acid, azelaic acid, or sebacic acid. One type of polyol component may be used alone or two or more used in combination. From the perspectives of adhesion to the reptile leather and adhesion to the surface layer, 1,4-butanediol, 1,6-hexanediol, or neopentyl glycol is favorably used as the alcohol and adipic acid is preferably used as the dibasic acid.

Examples of the polyisocyanate component of the polyester polyurethane include a known aliphatic, alicyclic, or aromatic organic isocyanate compound having at least two isocyanate groups in the molecule.

Examples of the aliphatic organic isocyanate compound include aliphatic diisocyanates such as butane-1,4-diisocyanate, hexamethylene diisocyanate, isopropylene diisocyanate, methylene diisocyanate, 2,2,4-trimethyl hexamethylene diisocyanate, lysine diisocyanate, and a dimer diisocyanate in which the carboxy group of a dimer acid is converted into an isocyanate group. Examples of the alicyclic organic isocyanate compound include alicyclic diisocyanates such as cyclohexane-1,4-diisocyanate, isophorone diisocyanate, norbornene diisocyanate, dicyclohexylmethane-4,4'-diisocyanate, 1,3-bis(isocyanatomethyl)cyclohexane, and methylcyclohexane diisocyanate. Examples of the aromatic organic isocyanate compound include aromatic diisocyanates such as 1,5-naphthylene diisocyanate, 4,4'-diphenylmethane diisocyanate, 4,4'-diphenyl dimethyl methane diisocyanate, 4,4'-dibenzyl diisocyanate, dialkyl diphenyl methane diisocyanate, tetraalkyl diphenyl methane diisocyanate, 1,3-phenylene diisocyanate, 1,4-phenylene diisocyanate, tolylene diisocyanate, xylylene diisocyanate, and m-tetramethyl xylylene diisocyanate. A tri- or polyfunctional polyisocyanate compound such as an adduct, an isocyanurate or a biuret may also be used in the polyisocyanate component. One type of polyisocyanate component may be used alone or two or more used in combination. From the perspectives of adhesion to the reptile leather and adhesion to the surface layer, an aliphatic diisocyanate and an aromatic diisocyanate are favorably used as the polyisocyanate component, and a hexamethylene diisocyanate and a tolylene diisocyanate are more favorably so used.

Although the average molecular weight of the polyester polyurethane is not particularly limited, the weight-average molecular weight is ordinarily 1,000 to 500,000.

The intermediate layer may contain other components within a scope that does not hinder the object of the present invention. Examples of the other components include an antioxidant and an ultraviolet absorber.

The thickness of the intermediate layer is not particularly limited but is set to a degree obtained by, for example, the manufacturing method described below.

The surface layer contains nitrocellulose. From the perspective of adhesion to the intermediate layer, the amount of nitrogen in nitrocellulose is ordinarily 10% to 13% and is preferably 10.7% to 12.2%. From the perspective of adhesion to the intermediate layer, the average degree of polymerization of the nitrocellulose is ordinarily 35 to 90 and is preferably 45 to 70.

The surface layer may contain other components within a scope that does not hinder the object of the present invention. Examples of the other components include resins such as a polyester resin and additives such as an antioxidant.

The thickness of the surface layer is not particularly limited but is set to a degree obtained by, for example, the manufacturing method described below.

In the first embodiment, a mixed layer containing polyester polyurethane and nitrocellulose is preferably formed between the intermediate layer and the surface layer. This configuration increases the adhesion of the intermediate layer and the surface layer. The intermediate layer preferably does not contain polyether polyurethane. In this case, the adhesion to the reptile leather and the adhesion to the surface layer increase.

Next the method for manufacturing a watchband, which is a reptile leather article of the first embodiment, is described.

The method for manufacturing the watchband comprises an intermediate layer formation step for forming an intermediate layer containing polyester polyurethane on the grain side of reptile leather, and a surface layer formation step for forming a surface layer containing nitrocellulose on the intermediate layer formed at the intermediate layer formation step.

Ordinarily a treatment for bringing out a luster is carried out as a finishing step of reptile leather. In the manufacture of the watchband, after the finishing step, steps are carried out such as a step for punching the reptile leather to obtain the leather with the shape and size required for the watchband body, a step for adjusting the thickness of the reptile leather, a lamination step for laminating pig leather or the like via a cushion layer, a core material layer or the like on the surface (the flesh side) opposite of the grain side of the reptile leather, and a hole-punching step for punching a hole for inserting the buckle tang according to need. The intermediate layer formation step and the surface layer formation step are ordinarily carried out after the lamination step and the hole-punching step, but the first embodiment is not limited thereto. A step for attaching the fastener or the like is carried out after the intermediate layer formation step and the surface layer formation step. In this manner, the watchband is manufactured through the intermediate layer formation step, the surface layer formation step and other known processing steps.

In detail, at the intermediate layer formation step, a composition containing polyester polyurethane is adhered to the grain side of the reptile leather treated to bring out a luster, and the result is dried to form the intermediate layer containing polyester polyurethane.

The composition containing polyester polyurethane contains the polyester polyurethane and a solvent. An organic solvent is suitably used as the solvent. Examples of the organic solvent include hydrocarbon solvents such as toluene and xylene, ketone solvents such as methyl ethyl ketone, ester solvents such as ethyl acetate, and ether solvents such as propylene glycol monomethyl ether. One of the solvents may be used alone or two or more used in combination. The composition containing polyester polyurethane may contain the other components that the intermediate layer may contain.

A commercially available composition (solution) may also be used for the composition containing polyester polyurethane. The concentration of the polyester polyurethane is appropriately adjusted for easy formation of the intermediate layer. The concentration of the polyester polyurethane is, for example, preferably 5 to 20 wt% inclusive and more preferably 7 to 9 wt% inclusive.

Examples of the method for applying the composition containing polyester polyurethane include brush application and spraying. The solvent in the composition that contains polyester polyurethane and adheres to the reptile leather is evaporated by setting the reptile leather ordinarily between 15 and 30 °C and preferably between 20 and 28 °C for 30 minutes, for example. This forms an intermediate layer containing polyester polyurethane on the grain side of the reptile leather.

In detail, at the surface layer formation step, the composition containing nitrocellulose is dried to form the surface layer containing nitrocellulose after being adhered onto the intermediate layer formed at the intermediate layer formation step.

The composition containing nitrocellulose contains the nitrocellulose and a solvent. An organic solvent is favorably used as the solvent. Examples of the organic solvent include the same solvents as for the composition containing polyester polyurethane and an alcohol solvent. Examples of the solvent include a hydrocarbon solvent such as toluene and xylene, a ketone solvent such as methyl ethyl ketone, an ester solvent such as ethyl acetate, an ether solvent such as propylene glycol monomethyl ether, and an alcohol solvent such as isopropyl alcohol and isobutyl alcohol. One of the solvents may be used alone or two or more used in combination. The composition containing nitrocellulose may contain the other components that the surface layer may contain. It is thought that the adhesion of the intermediate layer and the surface layer increases if the same solvent used in the composition containing polyester polyurethane is used in the composition containing nitrocellulose. This increased adhesion is thought to be due to a portion of the polyester polyurethane in the intermediate layer melting in the solvent when the composition containing nitrocellulose is adhered and a mixed layer containing polyester polyurethane and nitrocellulose forming between the intermediate layer and the surface layer.

A commercially available composition (e.g., a nitrocellulose lacquer) may be used as the composition containing nitrocellulose. The nitrocellulose may be used after adjusting the concentration, as appropriate, for easy formation of the surface layer. The concentration of the nitrocellulose is, for example, favorably 5 to 20 wt% inclusive and more favorably 9 to 13 wt% inclusive.

Examples of the method for applying the composition containing nitrocellulose include brush application and spraying. The solvent in the composition that contains nitrocellulose and adheres to the reptile leather is evaporated by setting the reptile ordinarily between 15 and 30 °C inclusive and preferably between 20 and 28 °C inclusive for 60 minutes, for example. The surface layer containing nitrocellulose is thereby formed on the intermediate layer.

The watchband thus obtained suppresses peeling of the surface layer containing nitrocellulose when the protective film pasted onto the fastener is peeled off.

### <Second embodiment>

The watchband, which is the reptile leather article of the second embodiment, is next described. The watchband differs from the reptile leather article of the first embodiment in that the reptile leather further contains a hexavalent chromium reducing compound component capable of reducing hexavalent chromium into trivalent chromium. Descriptions for features common to the reptile leather article of the first embodiment are omitted.

Reptile leather obtained through a chrome tanning step may contain harmful hexavalent chromium (a compound containing Cr(VI)). Therefore, the reptile leather may be treated with a hexavalent chromium treatment agent containing a hexavalent chromium reducing compound component. In this treatment, the hexavalent chromium reducing compound component reduces the hexavalent chromium to harmless trivalent chromium (a compound containing Cr(III)). The reptile leather after treatment contains, in addition to trivalent chromium, a residual hexavalent chromium reducing compound component that remained unused in the reduction. When the reptile leather is in this state, hexavalent chromium produced due to some cause after the treatment can be reduced to, for example, harmless trivalent chromium. In other words, the amount of hexavalent chromium can be kept below the limit specified by Regulation (EU) No. 3014/2014 (ordinarily less than 3 ppm) until the reptile leather article fulfills its function and purpose.

In detail, in the reptile leather article of the second embodiment, the reptile leather composing the watchband body is treated with a hexavalent chromium treatment agent. Therefore, the reptile leather composing the watchband body further contains a hexavalent chromium reducing compound component capable of reducing hexavalent chromium into trivalent chromium.

Examples of the hexavalent chromium reducing compound component include the component described in, for example, International Patent Application Laid-open No. 2016/021461. At least one type selected from the compound (A-i) represented by formula (A-i) and a tannin (A-ii) may also be suitably used as the hexavalent chromium reducing compound component.

The compound (A-i) is represented by formula (A-i) .

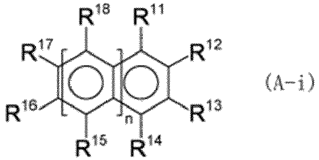

In the formula, n represents 0, 1, or 2. That is, the compound (A-i) has a benzene, naphthalene, or anthracene structure.

R¹¹ to R¹⁸ each independently represent a hydrogen atom, a hydroxy group, a C₁ to C₄ alkyl group, a C₁ to C₄ alkoxy group, or a group represented by formula (a-i). In formula (a-i), R¹⁹ represents a C₁ to C₄ alkyl group.

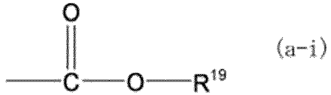

Examples of the C₁ to C₄ alkyl group include a methyl group, an ethyl group, an n-propyl group, an i-propyl group, an n-butyl group, an i-butyl group, an s-butyl group, and a t-butyl group. Examples of the C₁ to C₄ alkoxy group include a methoxy group, an ethoxy group, an n-propoxy group, an isopropoxy group, an n-butoxy group, an isobutoxy group, an s-butoxy group, and a t-butoxy group.

When n is 0, at least one of R¹¹ to R¹⁴, R¹⁶, and R¹⁷ is a hydroxy group. When n is 0, it is preferable that two or three of R¹¹ to R¹⁴, R¹⁶, and R¹⁷ be hydroxy groups because of the increased ability to reduce hexavalent chromium.

When n is 1 or 2, at least one of R¹¹ to R¹⁸ is a hydroxy group. When n is 1 or 2, it is preferable that two or three of R¹¹ to R¹⁸ be hydroxy groups because of the increased ability to reduce hexavalent chromium.

When n is 2, a plurality of R¹⁵ may be the same or different, and a plurality of R¹⁸ may be the same or different.

R¹⁶ and R¹⁷ may be linked together to form a five-membered ring or a six-membered ring. The atoms constituting the ring may include not only carbon atoms but also an oxygen atom. The ring may have a C₁ to C₁₆ alkyl group as a substituent. The C₁ to C₁₆ alkyl group may be linear or branched.

In the compound (A-i), n is 0, R¹⁴, R¹⁶, and R¹⁷ are hydroxy groups, and R¹² is suitably a gallic acid ester, which is the group resented by formula (a-i). Specific examples of the compound (A-i) include the compounds represented by formulas (1) to (10). The compound (A-i) may be used alone, or two or more compounds (A-i) may be used in combination.

- pyrogallol 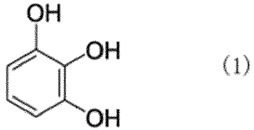
- gallic acid propyl 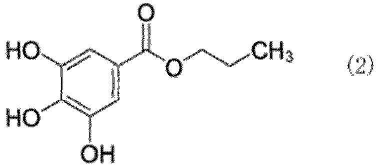
- 1,2,4-trihydroxybenzene 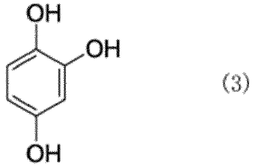
- fluoroglycinol 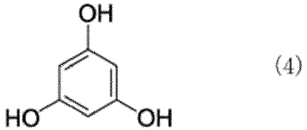
- resorcin

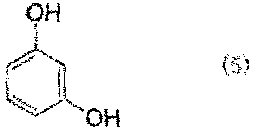

- hydroquinone 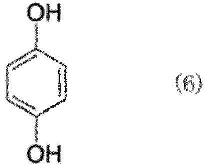
- catechol 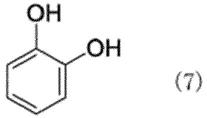
- 2,3-dihydroxynaphthalene 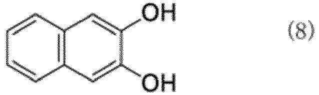
- 2,7-dihydroxynaphthalene 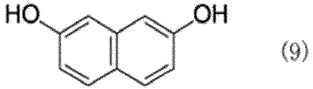
- 1,4,9,10-anthracenetetrol

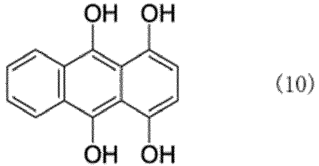

The tannin (A-ii) may be a hydrolyzable tannin or condensed tannin. Examples of the hydrolyzable tannin include gallotannins such as tannic acid (the compound represented by formula (11)) and ellagitannin. A hydrolyzable tannin is suitably used. The tannin (A-ii) may be used alone, or two or more tannins (A-ii) may be used in combination.

- tannic acid

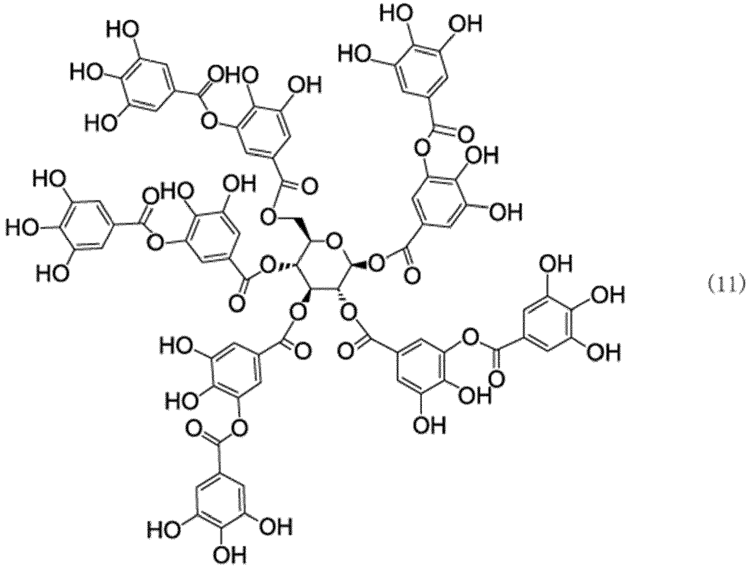

As the hexavalent chromium reducing compound component, at least one type selected from a compound (B-i) represented by formula (B-i) and a compound (B-ii) represented by formula (B-ii) is preferably used together with the compound (A-i) and the tannin (A-ii).

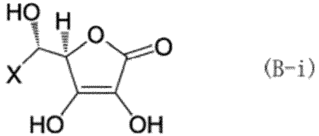

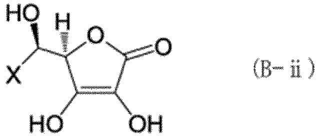

In the formulas above, X is a group represented by any one of formulas (b-i) to (b-iii). In formulas (b-i) to (b-iii), o represents an integer of 0 to 3, p represents an integer of 1 to 3, and q represents an integer of 1 to 17.

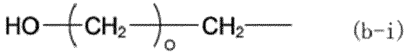

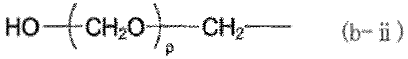

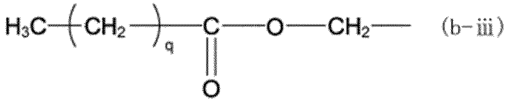

Specific examples of the compound (B-i) and compound (B-ii) include the compounds (ascorbic acid) represented by formula (12). The compound (B-i) may be used alone, or two or more compounds (B-i) may be used in combination. The compound (B-ii) may be used alone, or two or more compounds (B-ii) may be used in combination. The compounds (B-i) and (B-ii) may also be used in combination.

- ascorbic acid

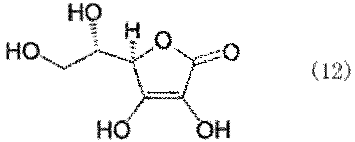

In particular, when the compound (A-i) which takes effect immediately and the tannin (A-ii) which takes effect slowly are combined, the hexavalent chromium content can be kept more reliably below the regulation limit until the reptile leather article fulfills its function and purpose. When the compound (B-i) and/or the tannin (B-ii) which has high reducing power and takes effect immediately is further combined with the compound (A-i) and/or the compound (A-ii), the hexavalent chromium in a reptile leather article, particularly that present near the surface, can be effectively reduced at the time of treatment. When coloring of the reptile leather is taken into consideration, the compound (A-i) is also preferably combined with the compound (B-i) and/or the compound (B-ii).

Next the method for manufacturing a watchband, which is a reptile leather article of the second embodiment, is described. The method for manufacturing a watchband differs from the first embodiment in that the reptile leather used in the intermediate layer formation step further contains a hexavalent chromium reducing compound component capable of reducing hexavalent chromium into trivalent chromium. Descriptions for features common to the reptile leather article of the first embodiment are omitted.

The reptile leather further containing a hexavalent chromium reducing compound component can be obtained by treating the reptile leather after the chrome tanning process with a hexavalent chromium treatment agent containing a hexavalent chromium reducing compound component. In the present specification, the step for treating with a hexavalent chromium treatment agent is referred to as the hexavalent chromium treatment step. Examples of the hexavalent chromium reducing compound component include the above-mentioned components and the hexavalent chromium reducing compound described in the specification of International Patent Application Laid-open No. 2016/21461. A treatment agent containing at least one type selected from the compound (A-i) and the tannin (A-ii) is preferably used as the hexavalent chromium reducing compound component. In addition to the compound (A-i) and the tannin (A-ii), a treatment agent containing one type selected from the compound (B-i) and the compound (B-ii) is also preferably used as the hexavalent chromium treatment agent.

The hexavalent chromium treatment step may ordinarily be carried out at any time after the chrome tanning step and before the intermediate layer formation step. Specifically, the hexavalent chromium treatment step may be carried out before, after or at the same time as the dyeing step and the fatliquoring step. The hexavalent chromium treatment step is preferably carried out before the treatment step for bringing out a luster.

The hexavalent chromium treatment step begins with the preparation of the hexavalent chromium treatment agent containing the hexavalent chromium reducing compound component and the solvent.

Examples of the solvent include water, a C₁ to C₃ alcohol (methanol, ethanol, propanol, and isopropanol (IPA)), butanol, acetone, methyl ethyl ketone (MEK), toluene, xylene, N,N-dimethylformamide (DMF), hexane, and heptane. The solvent may be used alone or two or more may be used in combination. Of these solvents, water alone or a mixed solvent of water and a C₁ to C₃ alcohol is preferably used and water alone or a mixed solvent of water and IPA is even more preferably used because the exterior appearance of the reptile leather is not easily damaged and because of the ease of handling.

The concentration of the hexavalent chromium reducing compound component in the hexavalent chromium treatment agent is adjusted, as appropriate, so that a favorably amount remains in the reptile leather after the hexavalent chromium treatment step.

Next, the chrome-tanned reptile leather is treated with the resulting hexavalent chromium treatment agent. Specifically, the hexavalent chromium treatment agent may be sprayed, sprinkled, or otherwise applied on the reptile leather for treatment, or the reptile leather may be dipped or immersed in the hexavalent chromium treatment agent for treatment. Also, a cloth impregnated with the hexavalent chromium treatment agent may be rubbed on the surface of the reptile leather for treatment. In this manner, the hexavalent chromium reducing compound component in the hexavalent chromium treatment agent can be impregnated into the reptile leather, rendering the hexavalent chromium harmless.

In this manner, the reptile leather containing the hexavalent chromium reducing compound component is used in the intermediate layer formation step in the manufacturing method of the second embodiment. Using an organic solvent as the solvent in the intermediate layer formation step and the surface layer formation step has the advantage of suppressing degeneration and extraction of the hexavalent chromium reducing compound component.

The resulting watchband suppresses peeling of the surface layer containing nitrocellulose when the protective film pasted onto the fastener is peeled off. Because the hexavalent chromium reducing compound component is not extracted from the reptile leather during manufacturing, the amount of hexavalent chromium can be kept below the limit specified by Regulation (EU) No. 3014/2014 (ordinarily less than 3 ppm) until the reptile leather article fulfills its function and purpose.

### <Other embodiments>

In the second embodiment, at least one type selected from the compound (A-i) and the tannin (A-ii) may be suitably used as the hexavalent chromium reducing compound component. In addition to the compound (A-i) and the tannin (A-ii), at least one type selected from the compound (B-i) and the compound (B-ii) is also more preferable. An example of the hexavalent chromium reducing compound component may be the component described in the specification of International Patent Application Laid-open No. 2016/021461. In contrast, in other embodiments, the hexavalent chromium reducing compound component is not particularly limited as long as the component can reduce hexavalent chromium into trivalent chromium. For example, the compound (B-i) or the compound (B-ii) may be used alone as the hexavalent chromium reducing compound component. More specifically, ascorbic acid may be used alone.

The reptile leather article in the first and second embodiments is a watchband. In contrast, in the other embodiments, the reptile leather article may be a reptile leather article other than a watchband. Descriptions for features common to the reptile leather article of the first or second embodiment are omitted.

Such a reptile leather article other than a watchband ordinarily has a main body composed of reptile leather, and a metal or other such part (e.g., a fastener or an ornament). Specific examples of the reptile leather article other than a watchband include a hat, a glove, a belt, a wallet, a business card holder, a briefcase, a bag, a book cover, a pencil case, a mobile phone case, a key case, a glasses case, and an accessory case. The reptile leather article of the other embodiments also contains reptile leather formed with the intermediate layer and the surface layer, and therefore exhibits the same effect. That is, peeling of the surface layer containing nitrocellulose is suppressed when the protective film pasted onto the metal or other such part is peeled off.

Such a reptile leather article is manufactured through known processing steps in addition to the steps described in the first and second embodiments.

In the reptile leather article in the first and second embodiments, an intermediate layer containing polyester polyurethane is provided on the grain side of the reptile leather treated to bring out the luster. In contrast, leather without treatment to bring out the luster may be used for the reptile leather in the other embodiments. Even if such leather is used, peeling of the surface layer containing nitrocellulose is suppressed when the protective film pasted onto the metal or other such part is peeled off.

Peeling of the surface layer being suppressed when the protective film is peeled off in the reptile leather article of the first and second embodiments was described as an example but the invention is not limited thereto. Peeling of the surface layer due to other causes can also be suppressed in the reptile leather article of the first and second embodiments.

As described above, the present invention relates to the following.
[1] The reptile leather article includes an intermediate layer containing polyester polyurethane and a surface layer containing nitrocellulose laminated, in that order, on the grain side of the reptile leather.
   The reptile leather article suppresses peeling of the surface layer containing nitrocellulose.
[2] The reptile leather article according to [1] includes the reptile leather containing a hexavalent chromium reducing compound component capable of reducing hexavalent chromium into trivalent chromium.
[3] The reptile leather article according to [2] includes the hexavalent chromium reducing compound component being at least one type selected from the compound (A-i) represented by formula (A-i) and the tannin (A-ii). 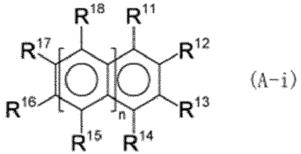
where n represents 0, 1, or 2, R¹¹ to R¹⁸ each independently represent a hydrogen atom, a hydroxy group, a C₁ to C₄ alkyl group, a C₁ to C₄ alkoxy group, or a group represented by the formula (a-i), where R¹⁹ represents a C₁ to C₄ alkyl group, at least one of R¹¹ to R¹⁴, R¹⁶, and R¹⁷ is a hydroxy group when n is 0, at least one of R¹¹ to R¹⁸ is a hydroxy group when n is 1 or 2, a plurality of R¹⁵ may be the same or different and a plurality of R¹⁸ may be the same or different when n is 2, R¹⁶ and R¹⁷ may be linked together to form a five-membered ring or a six-membered ring, and the ring may have a C₁ to C₁₆ alkyl group as a substituent.

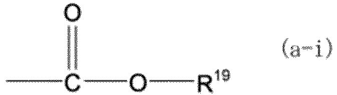

With the reptile leather article, the amount of hexavalent chromium can be kept below the limit specified by Regulation (EU) No. 3014/2014 (ordinarily less than 3 ppm) until the reptile leather article fulfills its function and purpose.

[4] The reptile leather article according to [3] includes the hexavalent chromium reducing compound component further containing at least one type selected from the compound (B-i) represented by formula (B-i) and the compound (B-ii) represented by formula (B-ii). 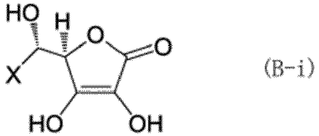 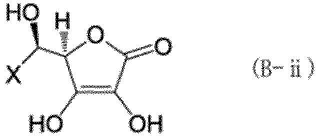 where X represents a group represented by any of formulas (b-i) to (b-iii), where o represents an integer of 0 to 3, p represents an integer of 1 to 3, and q represents an integer of 1 to 17.

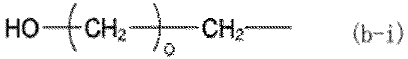

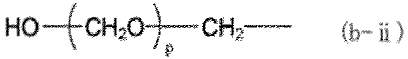

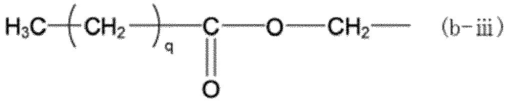

The reptile leather article keeps the amount of hexavalent chromium below the limit specified by Regulation (EU) No. 3014/2014 (ordinarily less than 3 ppm) until the reptile leather article fulfills its function and purpose.

[5] The reptile leather article according to any of [1] to [4] includes the reptile leather article being a watchband.

The reptile leather article is preferable in particular for maintaining the aesthetics.

[6] The method for manufacturing a reptile leather article includes an intermediate layer formation step for forming an intermediate layer containing polyester polyurethane on the grain side of reptile leather, and a surface layer formation step for forming a surface layer containing nitrocellulose on the intermediate layer formed at the intermediate layer formation step.

With the method for manufacturing the reptile leather article, peeling of the surface layer containing nitrocellulose can be suppressed in the resulting reptile leather article.

[7] The method for manufacturing a reptile leather article according to [6] includes the reptile leather containing a hexavalent chromium reducing compound component capable of reducing hexavalent chromium into trivalent chromium.
[8] The method for manufacturing a reptile leather article according to [7] includes the hexavalent chromium reducing compound component being at least one type selected from the compound (A-i) represented by formula (A-i) and the tannin (A-ii). 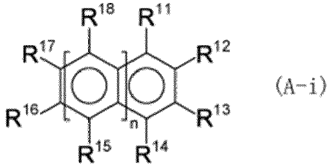 where n represents 0, 1, or 2, R¹¹ to R¹⁸ each independently represent a hydrogen atom, a hydroxy group, a C₁ to C₄ alkyl group, a C₁ to C₄ alkoxy group, or a group represented by the formula (a-i), where R¹⁹ represents a C₁ to C₄ alkyl group, at least one of R¹¹ to R¹⁴, R¹⁶, and R¹⁷ is a hydroxy group when n is 0, at least one of R¹¹ to R¹⁸ is a hydroxy group when n is 1 or 2, a plurality of R¹⁵ may be the same or different and a plurality of R¹⁸ may be the same or different when n is 2, R¹⁶ and R¹⁷ may be linked together to form a five-membered ring or a six-membered ring, and the ring may have a C₁ to C₁₆ alkyl group as a substituent.

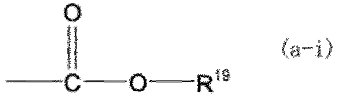

With the method for manufacturing a reptile leather article, the amount of hexavalent chromium can be kept below the limit specified by Regulation (EU) No. 3014/2014 (ordinarily less than 3 ppm) until the resulting reptile leather article fulfills its function and purpose.

[9] The method for manufacturing a reptile leather article according to [8] includes the hexavalent chromium reducing compound component further containing at least one type selected from the compound (B-i) represented by formula (B-i) and the compound (B-ii) represented by formula (B-ii). 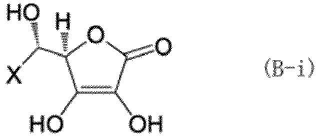 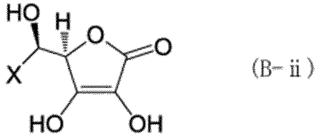 where X represents a group represented by any of formulas (b-i) to (b-iii), where o represents an integer of 0 to 3, p represents an integer of 1 to 3, and q represents an integer of 1 to 17.

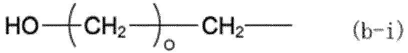

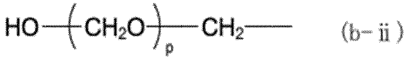

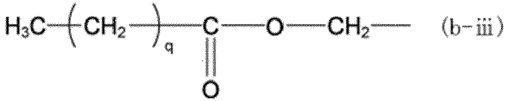

With the method for manufacturing a reptile leather article, the amount of hexavalent chromium can be more reliably kept below the limit specified by Regulation (EU) No. 3014/2014 (ordinarily less than 3 ppm) until the resulting reptile leather article fulfills its function and purpose.

[10] The method for manufacturing a reptile leather article according to any of [6] to [9] includes the reptile leather article being a watchband.

With the method for manufacturing a reptile leather article, the resulting reptile leather article is preferable because the aesthetics are maintained.

The present invention is described below in further detail on the basis of embodiments but the present invention is not limited thereto.

### [Examples]

### [Example 1-1]

A crocodile leather sheet that was glazed after chrome tanning was prepared.

A polyester polyurethane solution (1) was prepared containing a polyester polyurethane (1) with a concentration of 8 wt% formed by causing a polyester polyol made with polycondensing 1,4-butanediol, 1,6-hexanediol, neopentyl glycol and adipic acid to react with hexamethylene diisocyanate and tolylene diisocyanate. The solvent was organic.

A nitrocellulose lacquer containing nitrocellulose with a concetration of 10 wt% was also prepared. The solvent was organic.

The polyester polyurethane solution (1) was applied onto the leather sheet and the solvent was evaporated at 25 °C for 30 minutes. An intermediate layer was thereby formed on the leather sheet. Next the nitrocellulose lacquer was sprayed onto the intermediate layer and the solvent was evaporated at 25 °C for 60 minutes. A surface layer was thereby formed on the intermediate layer.

### [Example 1-2]

Other than using 3-methyl-1,5-pentanediol in place of the neopentyl glycol, a polyester polyurethane solution (2) was prepared in the same manner as in example 1-1.

Other than using the polyester polyurethane solution (2), an intermediate layer and a surface layer were formed on a leather sheet in the same manner as in example 1-1.

### [Example 1-3]

Other than using sebacic acid in place of adipic acid, a polyester polyurethane solution (3) was prepared in the same manner as in example 1-1.

Other than using the polyester polyurethane solution (3), an intermediate layer and a surface layer were formed on a leather sheet in the same manner as in example 1-1.

### [Example 1-4]

Other than using isophorone diisocyanate in place of the tolylene diisocyanate, a polyester polyurethane solution (4) was prepared in the same manner as in example 1-1.

Other than using the polyester polyurethane solution (4), an intermediate layer and a surface layer were formed on a leather sheet in the same manner as in example 1-1.

### [Examples 2-1 to 2-4]

Other than using a snake leather sheet that was glazed after chrome tanning in place of the crocodile leather sheet that was glazed after chrome tanning, an intermediate layer and a surface layer were formed on a leather sheet in the same manner as in examples 1-1 to 1-4.

### [Examples 3-1 to 3-4]

Other than using a lizard leather sheet that was glazed after chrome tanning in place of the crocodile leather sheet that was glazed after chrome tanning, an intermediate layer and a surface layer were formed on a leather sheet in the same manner as in examples 1-1 to 1-4.

### [Example 4]

A crocodile leather sheet that was glazed after chrome tanning was prepared.

The content of hexavalent chromium obtained by the technique of ISO 17075:2008-02 was 8 ppm in the leather sheet. When analyzed with a fluorescent X-ray analyzer (the JSX-3202EV Element Analyzer, an energy dispersive fluorescent X-ray analyzer manufactured by JEOL), the total chromium content was found to be 7141 ppm. The JSX3000 series reference sample 1, the JSX3000 series reference sample 2, and the JSX3000 series energy calibration reference sample manufactured by JEOL were used as reference samples. Measurements were made with a JSX starter and then PlasticD3 on the basis of the QuickManual (numbers EY07007-J00 and J00 EY07007G, August 2007 editions), which are JEOL materials.

Next, the hexavalent chromium treatment step was carried out on the leather sheet. 0.5 parts by mass of the compound indicated by formula (2), 2.5 parts by mass of the compound indicated by formula (11), and 2.0 parts by mass of the compound indicated by formula (12) were mixed and dissolved in water to obtain the hexavalent chromium treatment agent. The water was added so that the total amount of the treatment agent was 500 parts by mass. The resulting hexavalent chromium treatment agent was applied onto the leather sheet and dried.

After the drying, a portion of the leather was cut out and the hexavalent chromium content was found to be no greater than the detection limit (2 ppm) when measured with ISO 17075:2008-02. When the total chromium content was measured with a fluorescent X-ray analyzer, no change was found from before the hexavalent chromium treatment.

Other than using the leather sheet thus obtained, an intermediate layer and a surface layer were formed on the leather sheet in the same manner as example 1-1.

An organic solvent was used when preparing the polyester polyurethane solution (1) and the nitrocellulose lacquer used in the manfuacture of the leather sheet having an intermediate layer and a surface layer obtained in example 4. It is thought that it is because of the organic solvent use that the hexavalent chromium reducing compound component remained without change in the leather sheet obtained in example 4.

### [Comparative example 1]

Other than using a polyether polurethane solution in place of the polyester polyurethane solution (1), an intermediate layer was formed on a leather sheet in the same manner as in example 1-1. The intermediate layer containing polyether polyurethane appeared cloudy and lacked transparency.

### [Evaluation]

### [External observations]

The leather sheet having an intermediate layer and a surface layer obtained in the examples had luster. The intermediate layer and the surface layer were transparent. The intermediate layer obtained in comparative example 1 appeared cloudy and lacked transparency as described above.

### [Protective tape separation test]

A protective tape (3M Hyomen Hogo Teepu 332 (product name) manufactured by 3M Japan) 1 cm wide and 2 to 3 cm long was pasted onto each of the leather sheets having an intermediate layer and a surface layer obtained in the examples. Then the pasted protective tape was peeled off in one action.

The intermediate layer and the surface layer did not separate from the leather sheet having an intermediate layer and a surface layer obtained in the examples. In other words, there was no change in the luster from before the protective tape separation test.

## Claims

1. A reptile leather article comprising an intermediate layer containing polyester polyurethane and a surface layer containing nitrocellulose laminated, in that order, on a grain side of reptile leather.

2. The reptile leather article according to claim 1, wherein the reptile leather contains a hexavalent chromium reducing compound component capable of reducing hexavalent chromium into trivalent chromium.

3. The reptile leather article according to claim 2, wherein the hexavalent chromium reducing compound component is at least one type selected from a compound (A-i) represented by formula (A-i) and a tannin (A-ii): 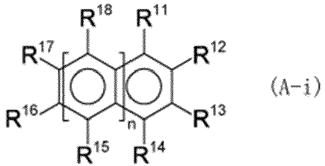 where, in formula (A-i), n represents 0, 1, or 2, R¹¹ to R¹⁸ each independently represent a hydrogen atom, a hydroxy group, a C₁ to C₄ alkyl group, a C₁ to C₄ alkoxy group, or a group represented by formula (a-i), where R¹⁹ represents a C₁ to C₄ alkyl group, at least one of R¹¹ to R¹⁴, R¹⁶, and R¹⁷ is a hydroxy group when n is 0, at least one of R¹¹ to R¹⁸ is a hydroxy group when n is 1 or 2, a plurality of R¹⁵ may be the same or different and a plurality of R¹⁸ may be the same or different when n is 2, R¹⁶ and R¹⁷ are optionally linked together to form a five-membered ring or a six-membered ring, and the ring optionally has a C₁ to C₁₆ alkyl group as a substituent:

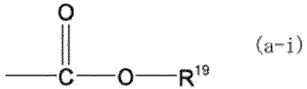

4. The reptile leather article according to claim 3, wherein the hexavalent chromium reducing compound component further contains at least one type selected from a compound (B-i) represented by formula (B-i) and a compound (B-ii) represented by formula (B-ii): 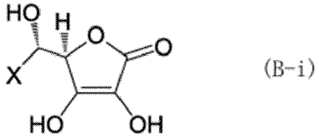 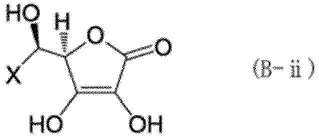 where X represents a group represented by any of formulas (b-i) to (b-iii), where o represents an integer of 0 to 3, p represents an integer of 1 to 3, and q represents an integer of 1 to 17:

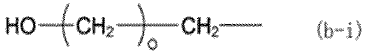

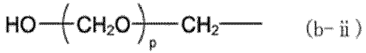

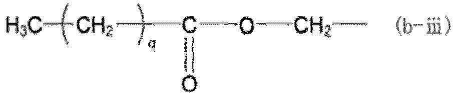

5. The reptile leather article according to any one of claims 1 to 4, wherein the reptile leather article is a watchband.

6. A method for manufacturing a reptile leather article, the method comprising an intermediate layer formation step for forming an intermediate layer containing polyester polyurethane on a grain side of reptile leather, and a surface layer formation step for forming a surface layer containing nitrocellulose on the intermediate layer formed at the intermediate layer formation step.

7. The method for manufacturing a reptile leather article according to claim 6, wherein the reptile leather contains a hexavalent chromium reducing compound component capable of reducing hexavalent chromium into trivalent chromium.

8. The method for manufacturing a reptile leather article according to claim 7, wherien the hexavalent chromium reducing compound component is at least one type selected from a compound (A-i) represented by formula (A-i) and a tannin (A-ii): 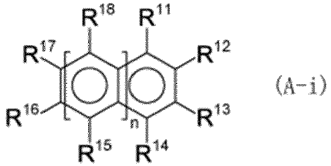 where, in formula (A-i), n represents 0, 1, or 2, R¹¹ to R¹⁸ each independently represent a hydrogen atom, a hydroxy group, a C₁ to C₄ alkyl group, a C₁ to C₄ alkoxy group, or a group represented by formula (a-i), where R¹⁹ represents a C₁ to C₄ alkyl group, at least one of R¹¹ to R¹⁴, R¹⁶, and R¹⁷ is a hydroxy group when n is 0, at least one of R¹¹ to R¹⁸ is a hydroxy group when n is 1 or 2, a plurality of R¹⁵ may be the same or different and a plurality of R¹⁸ may be the same or different when n is 2, R¹⁶ and R¹⁷ are optionally linked together to form a five-membered ring or a six-membered ring, and the ring optionally has a C₁ to C₁₆ alkyl group as a substituent:

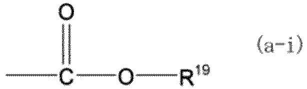

9. The method for manufacturing a reptile leather article according to claim 8, wherein the hexavalent chromium reducing compound component further contains at least one type selected from a compound (B-i) represented by formula (B-i) and a compound (B-ii) represented by formula (B-ii): 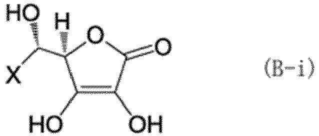 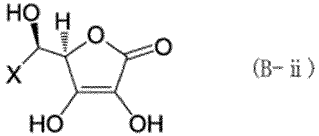 where X represents a group represented by any of formulas (b-i) to (b-iii), where o represents an integer of 0 to 3, p represents an integer of 1 to 3, and q represents an integer of 1 to 17:

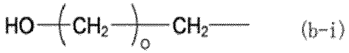

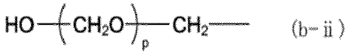

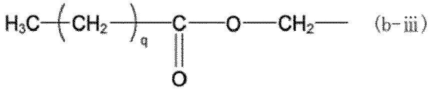

10. The method for manufacturing a reptile leather article according to any one of claims 6 to 9, wherein the reptile leather article is a watchband.

## Patentansprüche

1. Reptillederartikel, umfassend eine Zwischenschicht, enthaltend Polyesterpolyurethan, und eine Oberflächenschicht, enthaltend Nitrocellulose, die in dieser Reihenfolge auf eine Narbenseite des Reptilleders laminiert sind.

2. Reptillederartikel gemäß Anspruch 1, wobei das Reptilleder eine sechswertiges Chrom reduzierende Verbindungskomponente enthält, die in der Lage ist, sechswertiges Chrom in dreiwertiges Chrom zu reduzieren.

3. Reptillederartikel gemäß Anspruch 2, wobei die sechswertiges Chrom reduzierende Verbindungskomponente mindestens ein Typ ist, der aus einer Verbindung (A-i), dargestellt durch die Formel (A-i), und einem Tannin (A-ii) ausgewählt ist: 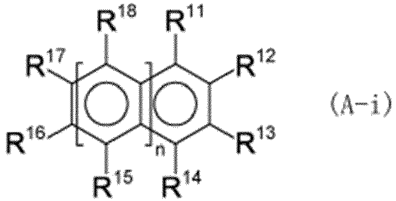 wobei in der Formel (A-i) n für 0, 1 oder 2 steht, R¹¹ bis R¹⁸ jeweils unabhängig voneinander für ein Wasserstoffatom, eine Hydroxygruppe, eine C₁- bis C₄-Alkylgruppe, eine C₁- bis C₄-Alkoxygruppe oder eine Gruppe, dargestellt durch die Formel (a-i), worin R¹⁹ für eine C₁- bis C₄-Alkylgruppe steht, stehen, mindestens eines von R¹¹ bis R¹⁴, R¹⁶ und R¹⁷ eine Hydroxygruppe ist, wenn n 0 ist, mindestens eines von R¹¹ bis R¹⁸ eine Hydroxygruppe ist, wenn n 1 oder 2 ist, mehrere R¹⁵ gleich oder verschieden sein können und mehrere R¹⁸ gleich oder verschieden sein können, wenn n 2 ist, R¹⁶ und R¹⁷ optional miteinander verbunden sind, um einen fünfgliedrigen Ring oder einen sechsgliedrigen Ring zu bilden, und der Ring optional eine C₁- bis C₁₆-Alkylgruppe als Substituent aufweist:

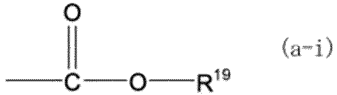

4. Reptillederartikel gemäß Anspruch 3, wobei die sechswertiges Chrom reduzierende Verbindungskomponente weiterhin mindestens einen Typ enthält, der aus einer Verbindung (B-i), dargestellt durch die Formel (B-i), und einer Verbindung (B-ii), dargestellt durch die Formel (B-ii), ausgewählt ist: 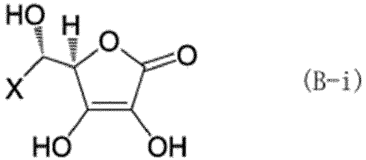 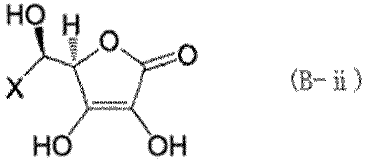 worin X für eine Gruppe steht, die durch eine der Formeln (b-i) bis (b-iii) dargestellt wird, worin o für eine ganze Zahl von 0 bis 3 steht, p für eine ganze Zahl von 1 bis 3 steht, und q für eine ganze Zahl von 1 bis 17 steht:

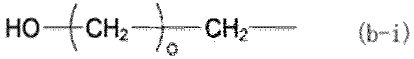

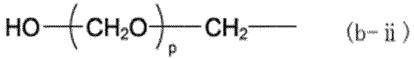

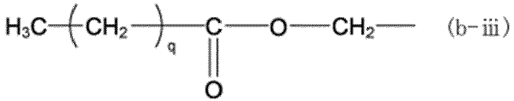

5. Reptillederartikel gemäß einem der Ansprüche 1 bis 4, wobei der Reptillederartikel ein Uhrenarmband ist.

6. Verfahren zur Herstellung eines Reptillederartikels, wobei das Verfahren einen Zwischenschichtbildungsschritt zum Bilden einer Polyesterpolyurethan enthaltenden Zwischenschicht auf einer Narbenseite des Reptilleders und einen Oberflächenschichtbildungsschritt zum Bilden einer Nitrocellulose enthaltenden Oberflächenschicht auf der in dem Zwischenschichtbildungsschritt gebildeten Zwischenschicht umfasst.

7. Verfahren zur Herstellung eines Reptillederartikels gemäß Anspruch 6, wobei das Reptilleder eine sechswertiges Chrom reduzierende Verbindungskomponente enthält, die in der Lage ist, sechswertiges Chrom in dreiwertiges Chrom zu reduzieren.

8. Verfahren zur Herstellung eines Reptillederartikels gemäß Anspruch 7, wobei die sechswertiges Chrom reduzierende Verbindungskomponente mindestens ein Typ ist, der aus einer Verbindung (A-i), dargestellt durch die Formel (A-i), und einem Tannin (A-ii) ausgewählt ist: 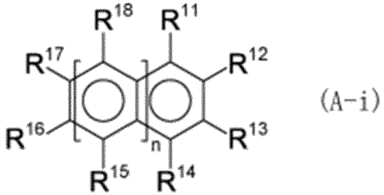 wobei in der Formel (A-i) n für 0, 1 oder 2 steht, R¹¹ bis R¹⁸ jeweils unabhängig voneinander für ein Wasserstoffatom, eine Hydroxygruppe, eine C₁- bis C₄-Alkylgruppe, eine C₁- bis C₄-Alkoxygruppe oder eine Gruppe, dargestellt durch die Formel (a-i), worin R¹⁹ für eine C₁- bis C₄-Alkylgruppe steht, stehen, mindestens eines von R¹¹ bis R¹⁴, R¹⁶ und R¹⁷ eine Hydroxygruppe ist, wenn n 0 ist, mindestens eines von R¹¹ bis R¹⁸ eine Hydroxygruppe ist, wenn n 1 oder 2 ist, mehrere R¹⁵ gleich oder verschieden sein können und mehrere R¹⁸ gleich oder verschieden sein können, wenn n 2 ist, R¹⁶ und R¹⁷ optional miteinander verbunden sind, um einen fünfgliedrigen Ring oder einen sechsgliedrigen Ring zu bilden, und der Ring optional eine C₁- bis C₁₆-Alkylgruppe als Substituent aufweist:

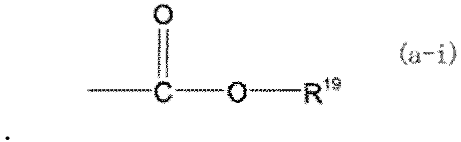

9. Verfahren zur Herstellung eines Reptillederartikels gemäß Anspruch 8, wobei die sechswertiges Chrom reduzierende Verbindungskomponente weiterhin mindestens einen Typ enthält, der aus einer Verbindung (B-i), dargestellt durch die Formel (B-i), und einer Verbindung (B-ii), dargestellt durch die Formel (B-ii), ausgewählt ist: 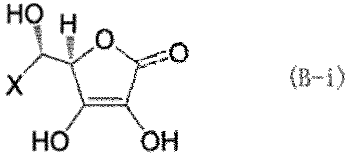 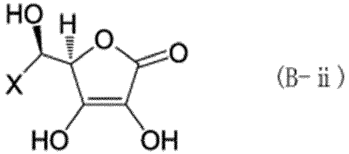 worin X für eine Gruppe steht, die durch eine der Formeln (b-i) bis (b-iii) dargestellt wird, worin o für eine ganze Zahl von 0 bis 3 steht, p für eine ganze Zahl von 1 bis 3 steht, und q für eine ganze Zahl von 1 bis 17 steht:

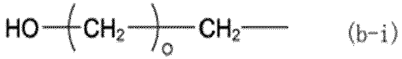

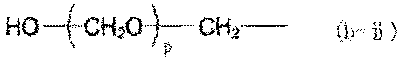

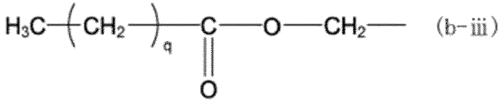

10. Verfahren zur Herstellung eines Reptillederartikels gemäß einem der Ansprüche 6 bis 9, wobei der Reptillederartikel ein Uhrenarmband ist.

## Revendications

1. Article en cuir de reptile comprenant une couche intermédiaire contenant un polyuréthane polyester et une couche de surface contenant une nitrocellulose stratifiée, dans cet ordre, sur un côté fleur de cuir de reptile.

2. Article en cuir de reptile selon la revendication 1, dans lequel le cuir de reptile contient un composant composé réducteur de chrome hexavalent capable de réduire le chrome hexavalent en chrome trivalent.

3. Article en cuir de reptile selon la revendication 2, dans lequel le composant composé réducteur de chrome hexavalent est au moins un type sélectionné parmi un composé (A-i) représenté par la formule (A-i) et un tanin (A-ii) : 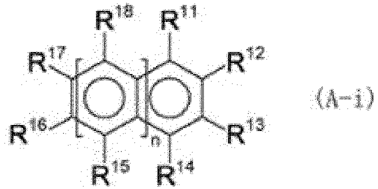 où, dans la formule (A-i), n représente 0, 1, ou 2, R¹¹ à R¹⁸ représentent chacun indépendamment un atome d'hydrogène, un groupe hydroxy, un groupe alkyle en C₁ à C₄, un groupe alcoxy en C₁ à C₄, ou un groupe représenté par la formule (a-i), où R¹⁹ représente un groupe alkyle en C₁ à C₄, au moins l'un de R¹¹ à R¹⁴, R¹⁶, et R¹⁷ est un groupe hydroxy quand n est 0, au moins l'un de R¹¹ à R¹⁸ est un groupe hydroxy quand n est 1 ou 2, une pluralité de R¹⁵ peuvent être identiques ou différents et une pluralité de R¹⁸ peuvent être identiques ou différents quand n est 2, R¹⁶ et R¹⁷ sont facultativement liés ensemble pour former un cycle à cinq chaînons ou un cycle à six chaînons, et le cycle présente facultativement un groupe alkyle en C₁ à C₁₆ en tant que substituant :

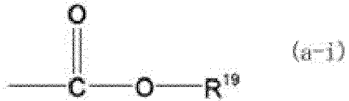

4. Article en cuir de reptile selon la revendication 3, dans lequel le composant composé réducteur de chrome hexavalent contient en outre au moins un type sélectionné parmi un composé (B-i) représenté par la formule (B-i) et un composé (B-ii) représenté par la formule (B-ii) : 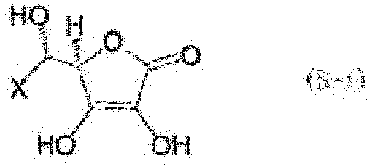 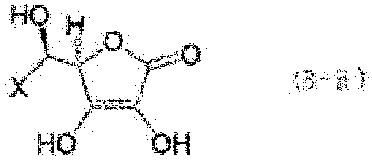 où X représente un groupe représenté par l'une quelconque des formules (b-i) à (b-iii), où o représente un nombre entier de 0 à 3, p représente un nombre entier de 1 à 3, et q représente un nombre entier de 1 à 17 :

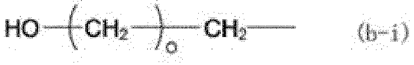

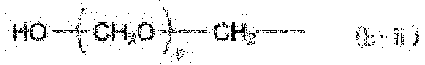

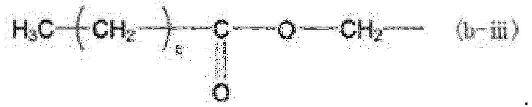

5. Article en cuir de reptile selon l'une quelconque des revendications 1 à 4, dans lequel l'article en cuir de reptile est un bracelet de montre.

6. Procédé de fabrication d'un article en cuir de reptile, le procédé comprenant une étape de formation de couche intermédiaire pour former une couche intermédiaire contenant un polyuréthane polyester sur un côté fleur du cuir de reptile, et une étape de formation de couche de surface pour former une couche de surface contenant une nitrocellulose sur la couche intermédiaire formée à l'étape de formation de couche intermédiaire.

7. Procédé de fabrication d'un article en cuir de reptile selon la revendication 6, dans lequel le cuir de reptile contient un composant composé réducteur de chrome hexavalent capable de réduire le chrome hexavalent en chrome trivalent.

8. Procédé de fabrication d'un article en cuir de reptile selon la revendication 7, dans lequel le composant composé réducteur de chrome hexavalent est au moins un type sélectionné parmi un composé (A-i) représenté par la formule (A-i) et un tanin (A-ii) : 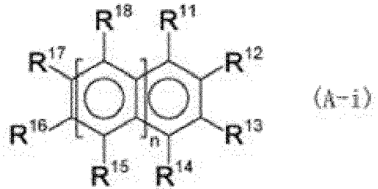 où, dans la formule (A-i), n représente 0, 1, ou 2, R¹¹ à R¹⁸ représentent chacun indépendamment un atome d'hydrogène, un groupe hydroxy, un groupe alkyle en C₁ à C₄, un groupe alcoxy en C₁ à C₄, ou un groupe représenté par la formule (a-i), où R¹⁹ représente un groupe alkyle en C₁ à C₄, au moins l'un de R¹¹ à R¹⁴, R¹⁶, et R¹⁷ est un groupe hydroxy quand n est 0, au moins l'un de R¹¹ à R¹⁸ est un groupe hydroxy quand n est 1 ou 2, une pluralité de R¹⁵ peuvent être identiques ou différents et une pluralité de R¹⁸ peuvent être identiques ou différents quand n est 2, R¹⁶ et R¹⁷ sont facultativement liés ensemble pour former un cycle à cinq chaînons ou un cycle à six chaînons, et le cycle présente facultativement un groupe alkyle en C₁ à C₁₆ en tant que substituant :

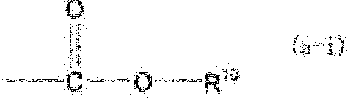

9. Procédé de fabrication d'un article en cuir de reptile selon la revendication 8, dans lequel le composant composé réducteur de chrome hexavalent contient en outre au moins un type sélectionné parmi un composé (B-i) représenté par la formule (B-i) et un composé (B-ii) représenté par la formule (B-ii) : 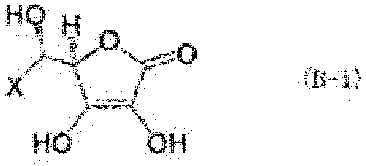 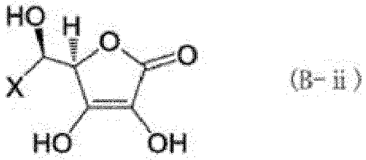 où X représente un groupe représenté par l'une quelconque des formules (b-i) à (b-iii), où o représente un nombre entier de 0 à 3, p représente un nombre entier de 1 à 3, et q représente un nombre entier de 1 à 17 :

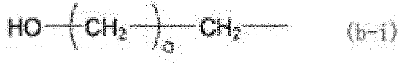

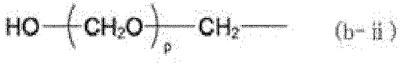

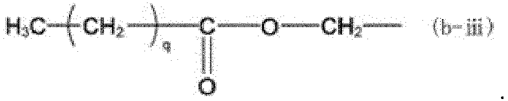

10. Procédé de fabrication d'un article en cuir de reptile selon l'une quelconque des revendications 6 à 9, dans lequel l'article en cuir de reptile est un bracelet de montre.
